# EUROPÄISCHE PATENTSCHRIFT

(11) **EP 3 676 183 B1**
(45) Veröffentlichungstag und Bekanntmachung des Hinweises auf die Patenterteilung: **13.01.2021**
(21) Anmeldenummer: 18765619.4
(22) Anmeldetag: 05.09.2018
(51) Int. Cl.: B65B 39/00, B65B 3/00, B65B 3/04, B65B 31/04

(54) **FÜLLNADEL ZUM ABGEBEN EINER PHARMAZEUTISCHEN FLÜSSIGKEIT IN EIN BEHÄLTNIS UND FÜLLEINRICHTUNG**
FILLING NEEDLE FOR DISPENSING A PHARMACEUTICAL FLUID IN A CONTAINER AND FILLING DEVICE
AIGUILLE DE REMPLISSAGE POUR DÉLIVRER UN FLUIDE MEDICAMENTEUX ET DISPOSITIF DE REMPLISSAGE

(30) Priorität: 08.11.2017 DE 102017219811
(43) Veröffentlichungstag der Anmeldung: 08.07.2020
(73) Patentinhaber: Syntegon Technology GmbH, 71332 Waiblingen (DE)
(72) Erfinder: KRAUSE, Bjoern, 74564 Crailsheim (DE)
(74) Vertreter: DREISS Patentanwälte PartG mbB
(86) Internationale Anmeldenummer: PCT/EP2018/073851
(87) Internationale Veröffentlichungsnummer: WO 2019/091622

(56) Entgegenhaltungen:
- DE-A1-102010 028 499
- DE-A1-102014 202 123
- US-A1- 2012 261 027
- US-A1- 2013 333 796
- US-A1- 2017 158 365

## Beschreibung

### Technisches Gebiet

Die Erfindung betrifft eine Füllnadel zum Abgeben einer pharmazeutischen Flüssigkeit in ein Behältnis sowie eine Fülleinrichtung mit wenigstens einer erfindungsgemäßen Füllnadel.

### Stand der Technik

Zum Abgeben bzw. Dosieren von pharmazeutischen Flüssigkeiten in Behältnisse wie Ampullen, Vials oder ähnlichem werden in der Regel Füllnadeln verwendet, die beispielsweise in die Kopfbereiche pharmazeutischen Behältnisse eintauchen können, indem die Füllnadeln an einer Fülleinrichtung heb- und senkbar angeordnet sind. Die Füllnadeln sind darüber hinaus über Füllschläuche mit einem Produktvorratsbehälter für die pharmazeutische Flüssigkeit verbunden, wobei es bekannt ist, das Dosieren einer bestimmten Menge an pharmazeutischer Flüssigkeit dadurch durchzuführen, dass die pharmazeutische Flüssigkeit im Produktvorratsbehälter unter Überdruck gesetzt ist und im Bereich der Füllnadel bzw. des zwischen der Füllnadel und dem Produktvorratstank angeordneten Füllschlauchs eine Ventileinrichtung, beispielsweise in Form eines Quetschventils, angeordnet ist. Den prinzipiellen Aufbau einer derartigen Fülleinrichtung ist aus der DE 10 2014 2002 123 A1 der Anmelderin bekannt. Weiterhin ist es aus der DE 10 2010 028 499 A1 der Anmelderin bekannt, eine Füllnadel dadurch auszubilden, dass zwei hülsenförmige Elemente einer Füllnadel koaxial zueinander angeordnet sind, wobei in dem Ringraum zwischen dem inneren und dem äußeren Element Gas strömt, welches eine Kontamination des Füllguts beim Befüllen vermeidet. Die pharmazeutische Flüssigkeit wird durch das zentral angeordnete Element in das Behältnis dosiert. US 2012/261027 A1, US 2013/333796 A1 und US 2017/158365 A1 beschreiben eine Füllnadel zum Abgeben einer pharmazeutischen Flüssigkeit in ein Behältnis, umfassend einen vorzugsweise stiftförmigen Füllnadelkörper, in dem eine Produktzuführbohrung ausgebildet ist, mit wenigstens einer, vorzugsweise mehreren Produktauslassbohrungen, die von der Produktzuführbohrung zumindest mittelbar ausgehen und an einer Außenwand des Füllnadelkörpers in einem in Bezug zu einer Längsachse des Füllnadelkörpers von einem Füllnadelkörperende beabstandeten Bereich münden.

Weiterhin ist es aus dem Stand der Technik auch bekannt, das Füllgut über Füllnadeln zu dosieren, die mit einem Kolbendosierer in Wirkverbindung angeordnet sind. Das Dosieren des Füllguts erfolgt somit über eine Ausschubbewegung eines Kolbens, wobei es weiterhin bekannt ist, durch eine am Dosierende stattfindende Rückwärtsbewegung des Kolbens ein Zurücksaugen des Füllguts in die Füllnadel zu bewirken, damit kein Füllgut nachtropft.

Die grundsätzliche Problematik bei pharmazeutischen Flüssigkeiten, insbesondere wenn diese eine relativ geringe Viskosität aufweisen, besteht darin, Fehldosierungen durch nachtropfende Flüssigkeit zu vermieden, da ein Zurückhalten der Flüssigkeit im Bereich der Füllnadel relativ schwierig ist bzw. ggf. relativ aufwendig ausgebildete Füllnadeln und Fülleinrichtungen benötigen.

### Offenbarung der Erfindung

Die erfindungsgemäße Füllnadel mit den Merkmalen des Anspruchs 1 hat den Vorteil, dass sie bei relativ einfachem konstruktivem Aufbau ein Nachtropfen von Füllgut aus der Füllnadel nach Ende des Dosiervorgangs sicher vermeidet. Dies wird erfindungsgemäß im Wesentlichen dadurch erreicht, dass die Füllnadel einen Füllnadelkörper aufweist, der zumindest im Bereich einer Produktauslassbohrung an dem Füllnadelkörper in Wirkverbindung bzw. Überdeckung mit einem elastischen Ventilglied angeordnet ist, das ab einem bestimmten Überdruck in einer Produktzuführbohrung bzw. an der Produktauslassbohrung den Strömungsweg für das flüssige Pharmazeutika in Richtung des pharmazeutischen Behältnisses freigibt, und das bei Unterschreitung des Überdrucks die Produktauslassbohrung verschließt und somit verhindert, dass Flüssigkeit aus der Produktauslassbohrung in Richtung des Behältnisses nachtropft bzw. nachströmt.

Vorteilhafte Weiterbildungen der erfindungsgemäßen Füllnadel zum Dosieren pharmazeutischer Flüssigkeiten sind in den Unteransprüchen aufgeführt.

Wenn der Querschnitt des Füllnadelkörpers zumindest im Bereich des Auslasses der wenigstens einen Produktauslassbohrung kreisförmig ausgebildet ist, wird es ermöglicht, einen (hülsenförmigen) Schlauchabschnitt als Ventilelement einzusetzen. Dieses dichtet dann den Bereich der Produktauslassbohrung deshalb besonders sicher und zuverlässig ab, weil das Ventilelement gleichmäßig am Außenumfang des Füllnadelkörpers anliegt und somit gleichmäßige bzw. homogene Zugspannungen in dem Material des Ventilelements erzeugt werden, die eine gleichmäßige Anlage des Ventilelements im Bereich des Auslasses der Produktauslassbohrung am Füllnadelkörper ermöglichen.

Um einen möglichst homogenen bzw. über den Querschnitt der Füllnadel gesehen gleichmäßigen Füllstrahl der Flüssigkeit zu ermöglichen, ist es vorgesehen, das bei Verwendung von mehreren Produktauslassbohrungen deren Auslässe in der Außenwand des Füllnadelkörpers in gleichmäßigen Winkelabständen um die Längsachse des Füllnadelkörpers münden.

Zur Erhöhung der Durchflussmenge bei einem bestimmten Überdruck der Flüssigkeit und begrenztem Querschnitt der Produktauslassbohrung kann es weiterhin zur räumlich vorteilhaften Anordnung der Produktauslassbohrungen vorgesehen sein, dass die Auslässe der Produktauslassbohrungen in Bezug auf die Längsachse des Füllnadelkörpers in wenigstens zwei unterschiedlichen Bereichen in der Außenwand des Füllnadelkörpers münden.

Hinsichtlich der axialen Länge und genauen Position des (schlauchförmigen) Ventilelements in Bezug auf das dem pharmazeutischen Behältnis zugewandte Ende des Füllnadelkörpers gibt es grundsätzlich zwei verschiedene Möglichkeiten: Bei einer ersten Ausgestaltung ist das Ventilelement hülsenförmig ausgebildet, wobei es das Füllnadelkörperende in Richtung der Längsachse betrachtet überragt. Bei einer derartigen Ausführungsform wird insbesondere sichergestellt, dass sich gegebenenfalls im Bereich des Füllnadelkörperendes vorhandene Flüssigkeit am Innenumfang des Ventilelements ansammeln kann. Alternativ hierzu ist es jedoch bei einer zweiten Ausführungsform vorgesehen, dass das Ventilelement hülsenförmig ausgebildet ist, dass das Füllnadelkörperende konisch ausgebildet ist, und dass das Ventilelement in Richtung der Längsachse betrachtet in dem konischen Bereich endet. Eine derartige Ausgestaltung hat insbesondere den Vorteil, dass sich das eine stirnseitige Ende des Ventilelements im Bereich des konischen Füllnadelkörperendes an den Füllnadelkörper anlegen kann bzw. sich an dieses anschmiegt. Somit wird beim Freigeben ein im Querschnitt reduzierter Füllstrahl erzeugt, der darüber hinaus den Vorteil aufweist, dass das Produkt verwirbelungsfrei bzw. laminar von der Füllnadel abströmt, wodurch auch Lufteinschlüsse im Produkt bzw. im zu befüllenden Behälter vermieden werden.

Eine weitere konstruktive Ausgestaltung des Füllnadelkörpers sieht vor, dass eine radial um die Längsachse des Füllnadelkörpers umlaufende Querschnittsreduzierung vorgesehen ist. Eine derartige Ausgestaltung hat insbesondere den Vorteil, dass beim Rücksaugen von Flüssigkeit sich das Ventilelement im Bereich der Querschnittsreduzierung an den Füllnadelkörper anschmiegen kann und dadurch für eine besonders gute Abdichtwirkung sorgt.

Hierbei kann in Weiterbildung des zuletzt gemachten Vorschlags insbesondere vorgesehen sein, dass die Querschnittsreduzierung einen gerundet ausgebildeten Querschnitt aufweist. Die hat insbesondere den Vorteil, dass sich das Ventilelement spaltfrei an die Außenwand des Füllnadelkörpers anschmiegen bzw. anlegen kann.

Um das Ventilelement mit dem Füllnadelkörper zu verbinden, findet bevorzugt ein separates Befestigungselement oder eine Formschlussverbindung an dem Füllnadelkörper Verwendung. Diese Befestigungsarten ermöglichen es darüber hinaus, dass ein Austausch des Ventilelements auf relativ einfache Art und Weise möglich ist, und dass das Ventilelement besonders sicher am Füllnadelkörper gehalten ist.

Eine weitere Ausgestaltung der Erfindung betrifft eine Fülleinrichtung mit wenigstens einer soweit beschriebenen erfindungsgemäßen Füllnadel, wobei sich die Fülleinrichtung dadurch auszeichnet, dass Mittel zum Rücksaugen von Flüssigkeit in den Füllnadelkörper vorgesehen sind. Durch das Rücksaugen von Flüssigkeit in den Füllnadelkörper beim Dosierende wird zum einen ein weiterer Austritt von Flüssigkeit aus dem Füllnadelkörper in Richtung des pharmazeutischen Behältnisses sicher vermieden, zum anderen wird durch den dadurch erzeugten Unterdruck das Ventilelement gegen den Auslass der Produktauslassbohrung angelegt, wodurch eine besonders sichere und zuverlässige Abdichtung der Produktauslassbohrung am Füllnadelkörper erzielbar ist.

Weitere Vorteile, Merkmale und Einzelheiten der Erfindung ergeben sich aus der nachfolgenden Beschreibung bevorzugter Ausführungsbeispiele sowie anhand der Zeichnung.

Diese zeigt in:
- Fig. 1: eine vereinfachte Darstellung einer Fülleinrichtung zum Dosieren von flüssigen Pharmazeutika in Behältnisse und
- Fig. 2 bis Fig. 8: jeweils ein einem Behältnis zugewandter Endbereich einer Füllnadel in unterschiedlichen konstruktiven Ausgestaltungen.

Gleiche Elemente bzw. Elemente mit gleicher Funktion sind in den Figuren mit den gleichen Bezugsziffern versehen.

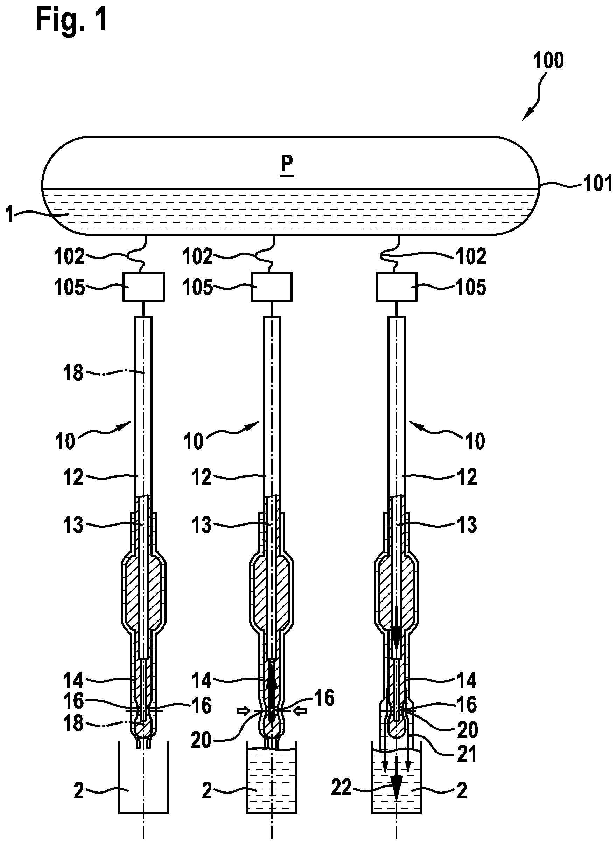

In der Fig. 1 ist eine Fülleinrichtung 100 zum Abgeben bzw. Dosieren einer pharmazeutischen Flüssigkeit 1 in pharmazeutische Behältnisse 2, beispielsweise Ampullen, Vials oder ähnlichem stark vereinfacht dargestellt. Bei der pharmazeutischen Flüssigkeit 1 handelt es sich insbesondere um eine niedrigviskose oder eine relativ geringe Oberflächenspannung bzw. kriechfähige aufweisende Flüssigkeit 1, insbesondere auf Öl- oder Alkoholbasis.

Die Fülleinrichtung 100 umfasst einen Produktvorratstank 101 für die Flüssigkeit 1, in der die Flüssigkeit 1 beispielsweise unter einem bestimmten Überdruck P gespeichert ist. Von dem Produktvorratstank 101 gehen mehrere Füllschläuche 102 aus, die beispielhaft unter Zwischenschaltung jeweils eines Kolbendosierers 105 mit jeweils einer erfindungsgemäßen Füllnadel 10 verbunden sind. Der Kolbendosierer 105 ist dazu ausgebildet, eine bestimmte Menge an Flüssigkeit 1 entsprechend eines Vorschubwegs bzw. Hubs eines Kolbens in Richtung der Füllnadel 10 zu fördern, und bei einer Rückwärtsbewegung des in der Fig. 1 nicht dargestellten Kolbens Flüssigkeit 1 aus der Füllnadel 10 zurückzusaugen.

Jede Füllnadel 10 weist im Wesentlichen einen vorzugsweise aus Metall bestehenden, stiftförmigen bzw. zylindrischen Füllnadelkörper 12 auf, der auf der dem Behältnis 2 zugewandten Endbereich radial von einem hülsenförmigen, aus einem elastischen Material wie einem Elastomer ausgebildeten, schlauchartigen Ventilelement 14 umgeben ist. In dem Füllnadelkörper 12 ist eine Produktzuführbohrung 13, beispielhaft in Form einer Sacklochbohrung ausgebildet, von der vorzugsweise mehrere radial nach außen ragende Produktauslassbohrungen 16 in gleichmäßigen Winkelabständen um die Längsachse 18 des Füllnadelkörpers 12 ausgehen, die an der Außenfläche des Füllnadelkörpers 12 im Überdeckungsbereich mit dem Ventilelement 14 münden.

Ergänzend wird erwähnt, dass der Füllnadelkörper 12, wie an sich aus dem Stand der Technik bekannt und üblich, auch als Rohr ausgebildet sein kann, das im Bereich des dem Behältnis 2 zugewandten Endbereichs mit einem Verschlussstück versehen ist, das in die Öffnung des Rohrs dichtend eingesetzt ist.

Weiterhin wird erwähnt, dass das Ventilelement 14 entweder aus einem produktabweisenden Material ausgebildet sein kann, oder aber zumindest auf der dem Füllnadelkörper 12 zugewandten (Innen-) Seite mit einer entsprechenden Beschichtung versehen ist, um ein Anhaften von Produkt zwischen dem Ventilelement 14 und dem Füllnadelkörper 12 zu erschweren bzw. zu vermeiden.

Unter Produktauslassbohrungen 16 im Sinne der Erfindung sollen nicht nur Bohrungen im engeren Sinne (d.h. Öffnungen mit runder Querschnittsfläche) verstanden werden, sondern alle Produktauslassöffnungen, unabhängig von deren Querschnittsform oder der Art, wie sie erzeugt wurden, beispielsweise durch eine Laserstrahleinrichtung.

In der Fig. 1 sind der Einfachheit halber und zur Verdeutlichung der Funktion des Ventilelements 14 drei Füllnadeln 10 während unterschiedlicher Dosierphasen der Flüssigkeit 1 dargestellt. Selbstverständlich liegt es im Rahmen der Erfindung, dass die Fülleinrichtung 100 mehr als drei Füllnadeln 10 aufweist, die beispielsweise in jeweils gleichmäßigen Abständen zueinander angeordnet sind, um die unterhalb der Füllnadeln 10 taktweise mittels einer nicht gezeigten Fördereinrichtung geförderten Behältnisse 2 zu befüllen. Weiterhin werden beim Füllbetrieb die Füllnadeln 10 gemeinsam bzw. gleich angesteuert, sodass die nachfolgend kurz erläuterten drei Dosierphasen jeweils an allen Füllnadeln 10 der Fülleinrichtung 100 gleichzeitig stattfinden.

Die in der Fig. 1 links dargestellte Füllnadel 10 ist in einem Betriebszustand der Fülleinrichtung 100 gezeigt, bei der das Ventilelement 14 die am Füllnadelkörper 12 ausgebildeten Produktauslassbohrungen 16 verschließt, um ein Abgeben von Flüssigkeit 1 in das Behältnis 2 zu vermeiden. Dabei herrscht im Bereich den Produktauslassbohrungen 16 maximal lediglich ein relativ geringer Überdruck, der eine Abdichtung zwischen dem Füllnadelkörper 12 und dem Ventilelement 14 gegen einen Austritt von Flüssigkeit 1 in Richtung des Behältnisses 2 sicherstellt.

Bei der in der Fig. 1 mittleren Füllnadel 10 ist demgegenüber der Zustand dargestellt ist, bei der mittels des Kolbendosierers 105 ein Rücksaugen von Flüssigkeit 1 aus der Füllnadel 10 stattfindet. In diesem Fall schmiegt sich das Ventilelement 16 im Bereich einer radial an dem Füllnadelkörper 12 um eine Längsachse 18 ausgebildete Querschnittsverjüngung 20, die im Bereich der wenigstens einen Produktauslassbohrung 16 angeordnet ist, an.

Zuletzt zeigt die in der Fig. 1 rechte Füllnadel 10 den Füllbetrieb, bei der mittels des Kolbendosierers 105 in dem Füllnadelkörper 12 bzw. der Produktauslassbohrung 16 die Flüssigkeit 1 einen Überdruck aufweist, der hoch genug ist, damit das Ventilelement 14 im Bereich der Produktauslassbohrung 16 radial aufgedehnt wird. Dabei gelangt die Flüssigkeit 1 entsprechend der Pfeile 21 in den radialen Ringraum zwischen dem Füllnadelkörper 12 und dem Innenumfang des Ventilelements 14 und strömt am Ende des Füllnadelkörpers 12 in Richtung des Behältnisses 2, was durch den Pfeil 22 dargestellt sein soll.

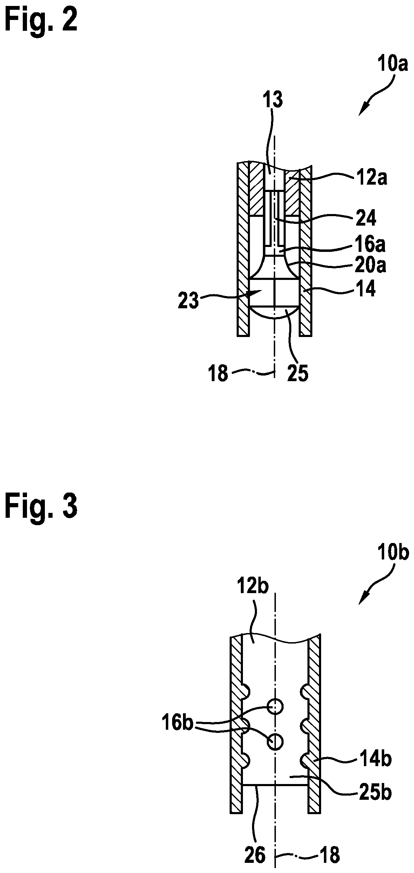

In der Fig. 2 ist abschnittsweise eine Füllnadel 10a dargestellt, bei der die Querschnittsverjüngung 20a im Bereich eines separaten, als Verschlussstopfen dienenden Bauteils 23 für den rohrförmigen Füllnadelkörper 12a ausgebildet ist. Die Produktzulaufbohrung 13 in dem Füllnadelkörper 12a hat Verbindung mit einer Sacklochbohrung 24 im Bauteil 23. Von der Sacklochbohrung 24 gehen wiederum die Produktauslassbohrungen 16a aus. Weiterhin weist die Querschnittsverjüngung 20a einen zumindest bereichsweise gerundet ausgebildeten Querschnitt auf. Die Querschnittsverjüngung 20a bewirkt eine gleichmäßigere Verteilung der Flüssigkeit am Innenumfang des Ventilelements 14. Weiterhin ist das Füllnadelkörperende 25 gerundet bzw. ballig ausgebildet, und das aus einem elastischen Material bestimmte Ventilelement 14 überragt das Füllnadelkörperende 25 in Richtung der Längsachse 18 in axialer Richtung. Ergänzend wird erwähnt, dass die Produktauslassbohrungen 16a nicht senkrecht zur Längsachse 18 verlaufen müssen, sondern beispielsweise auch in einem schrägen Winkel angeordnet sein können.

In der Fig. 3 ist eine Füllnadel 10b dargestellt, bei der in Bezug auf die Längsachse 18 in wenigstens zwei axial voneinander beabstandeten Bereichen jeweils mehrere Produktauslassbohrungen 16b vorgesehen sind. Die Produktauslassbohrungen 16b sind vorzugsweise in gleichmäßigen Winkelabständen um die Längsachse 18 des Füllnadelkörpers 12b angeordnet. Beispielhaft ist das Füllnadelkörperende 25b mit einer senkrecht zur Längsachse 18 verlaufenden Stirnseite 26 ausgebildet. Auch bei der Füllnadel 10b findet eine besonders gleichmäßige Verteilung der Flüssigkeit 1 am Innenumfang des Ventilelements 14b statt.

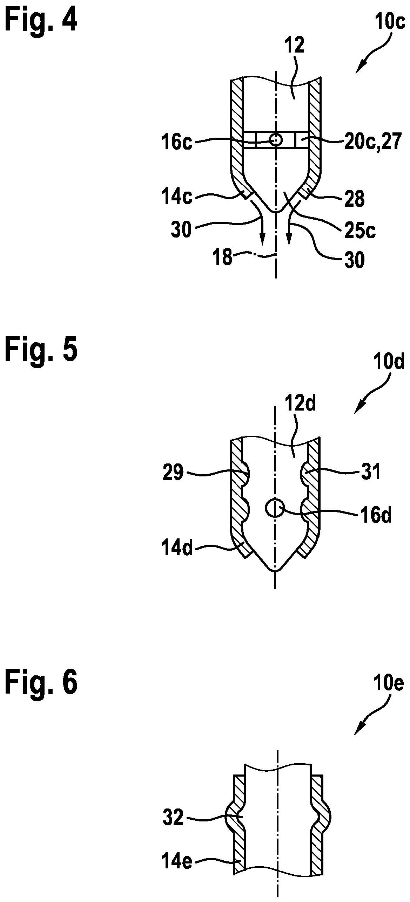

Bei der in der Fig. 4 dargestellten Füllnadel 10c weist diese ein in etwa konisch bzw. kegelförmig ausgebildetes Füllnadelkörperende 25c auf. Weiterhin sind die Produktauslassbohrungen 16c im Bereich einer Querschnittsverengung 20c ausgebildet, die als eine einen rechteckigen Querschnitt aufweisende Ringnut 27 ausgebildet ist. Das Ventilelement 14c überragt das Füllnadelkörperende 25c nicht, sondern endet in axialer Richtung betrachtet kurz vor diesem, sodass aufgrund der unter elastischer Vorspannung an der Außenwand des Füllnadelkörpers 12c anliegende Ventilelement 14c ein Endbereich 28 des Ventilelements 14c sich in radialer Richtung in Bezug auf die Längsachse 18 an das Füllnadelkörperende 25c anschmiegt. Beim Abgeben der Flüssigkeit 1 aus dem Bereich des Füllnadelkörperendes 25c wird die Flüssigkeit 1 radial besser zusammengeführt bzw. weist der Füllstrahl einen geringeren Querschnitt auf, was durch die Pfeile 30 verdeutlicht sein soll.

In der Fig. 5 ist eine Füllnadel 10d dargestellt, die unmittelbar oberhalb der Produktauslassbohrungen 16d eine radial umlaufende Ringnut 29 aufweist. In diese Ringnut 29 ragt eine radial umlaufende Verdickung 31 des Ventilelements 14d formschlüssig ein, um das Ventilelement 14d an dem Füllnadelkörper 12d zu positionieren bzw. zu fixieren. Durch den geringen axialen Abstand zwischen der Befestigung des Ventilelements 14d und den Produktauslassbohrungen 16d ergibt sich eine geringere Produktnachlaufmenge.

Demgegenüber weist die Füllnadel 10e entsprechend der Fig. 6 eine radial umlaufende Verdickung 32 auf, in deren Bereich das Ventilelement 14e unter erhöhter Umfangsspannung an dem Füllnadelkörper 14e gehalten ist. Durch die Verdickung 32 findet eine Abdichtung beidseitig der Verdickung 32 durch das Ventilelement 14e statt.

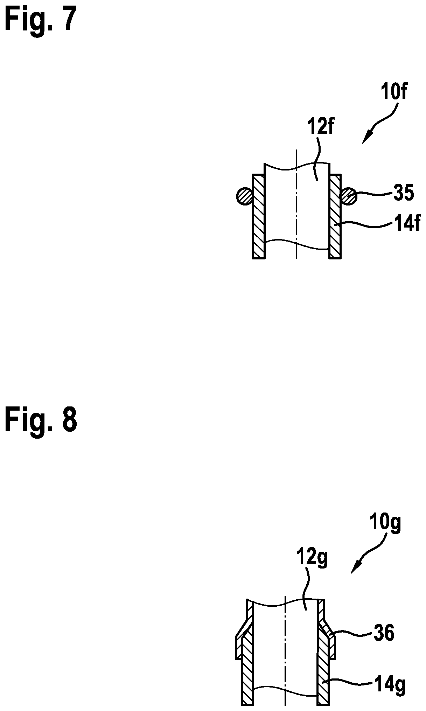

Bei der Füllnadel 10f entsprechend der Fig. 7 ist das Ventilelement 14f durch ein separates Bauteil 35, beispielsweise in Form eines O-Rings oder eines Klemmrings, am Außenumfang des Füllnadelkörpers 12f fixiert.

In ähnlicher Weise ist zuletzt in der Fig. 8 eine Füllnadel 10g dargestellt, bei der ein Klemmring 36, der sich in axialer Richtung betrachtet über das Ventilelement 14g hinaus erstreckt, der Fixierung bzw. Befestigung des Ventilelements 14g an dem Füllnadelkörper 12g dient.

## Patentansprüche

1. Füllnadel (10; 10a bis 10g) zum Abgeben einer pharmazeutischen Flüssigkeit (1) in ein Behältnis (2), umfassend einen vorzugsweise stiftförmigen Füllnadelkörper (12; 12a; 12b; 12d; 12f; 12g), in dem eine Produktzuführbohrung (13) ausgebildet ist, mit wenigstens einer, vorzugsweise mehreren Produktauslassbohrungen (16; 16a bis 16d), die von der Produktzuführbohrung (13) zumindest mittelbar ausgehen und an einer Außenwand des Füllnadelkörpers (12; 12a; 12b; 12d; 12f; 12g) in einem in Bezug zu einer Längsachse (18) des Füllnadelkörpers (12; 12a; 12b; 12d; 12f; 12g) von einem Füllnadelkörperende (25; 25b; 25c) beabstandeten Bereich münden, und mit einem den Füllnadelkörper (12; 12a; 12b; 12d; 12f; 12g) zumindest im Bereich der wenigstens einen Produktauslassbohrung (16; 16a bis 16d) überdeckenden, aus einem elastischen Material bestehenden Ventilelement (14; 14b; 14c; 14d; 14e; 14g), das ab einem bestimmten Überdruck in der Produktauslassbohrung (16; 16a bis 16d) die wenigstens eine Produktauslassbohrung (16; 16a bis 16d) zum Abgeben von Flüssigkeit (1) freigibt und bei Unterschreitung des bestimmten Überdrucks die wenigstens eine Produktauslassbohrung (16; 16a bis 16d) verschließt.

2. Füllnadel nach Anspruch 1,
**dadurch gekennzeichnet,**
**dass** der Querschnitt des Füllnadelkörpers (12; 12a; 12b; 12d; 12f; 12g) zumindest im Bereich der wenigstens einen Produktauslassbohrung (16; 16a bis 16d) kreisförmig ausgebildet ist.

3. Füllnadel nach Anspruch 1 oder 2,
**dadurch gekennzeichnet,**
**dass** bei Verwendung von mehreren Produktauslassbohrungen (16; 16a bis 16d) deren Auslässe in der Außenwand des Füllnadelkörpers (12; 12a; 12b; 12d; 12f; 12g) in gleichmäßigen Winkelabständen um die Längsachse (18) münden.

4. Füllnadel nach einem der Ansprüche 1 bis 3,
**dadurch gekennzeichnet,**
**dass** bei Verwendung von mehreren Produktauslassbohrungen (16b) deren Auslässe in Bezug auf die Längsachse (18) in wenigstens zwei unterschiedlichen Bereichen in der Außenwand des Füllnadelkörpers (12b) münden.

5. Füllnadel nach einem der Ansprüche 1 bis 4,
**dadurch gekennzeichnet,**
**dass** das Ventilelement (14; 14b) hülsenförmig ausgebildet ist und das Füllnadelkörperende (25; 25b) in Richtung der Längsachse (18) betrachtet überragt.

6. Füllnadel nach einem der Ansprüche 1 bis 4,
**dadurch gekennzeichnet,**
**dass** das Ventilelement (14c; 14d) hülsenförmig ausgebildet ist, dass das Füllnadelkörperende (25c) konisch ausgebildet ist, und dass das Ventilelement (14c; 14d) in Richtung der Längsachse (18) betrachtet in dem konischen Bereich des Füllnadelkörperendes (25c) endet.

7. Füllnadel nach einem der Ansprüche 1 bis 6,
**dadurch gekennzeichnet,**
**dass** der wenigstens eine Auslass einer Produktauslassbohrung (16c) im Bereich einer radial um die Längsachse (18) umlaufenden Querschnittsverjüngung (20c) des Füllnadelkörpers (12) ausgebildet ist.

8. Füllnadel nach Anspruch 7,
**dadurch gekennzeichnet,**
**dass** die Querschnittsverjüngung (20c) in Form einer Ringnut (27) ausgebildet ist.

9. Füllnadel nach einem der Ansprüche 1 bis 6,
**dadurch gekennzeichnet,**
**dass** die wenigstens eine Produktauslassbohrung (16a) in einem von dem Füllnadelkörper (12) separaten, als Verschluss für den Füllnadelkörper (12) dienenden Bauteil (23) ausgebildet ist, dass der Auslass der wenigstens einen Produktauslassbohrung (16a) im Bereich einer radial um die Längsachse (18) umlaufenden Querschnittsverjüngung (20a) des Bauteils (23) ausgebildet ist, und dass die Querschnittsverjüngung (20a) einen gerundeten Querschnitt aufweist.

10. Füllnadel nach einem der Ansprüche 1 bis 9,
**dadurch gekennzeichnet,**
**dass** das Ventilelement (14; 14b; 14c; 14d; 14e; 14g) durch ein separates Befestigungselement (36; 36) oder eine Formschlußverbindung an dem Füllnadelkörper (12; 12a; 12b; 12d; 12f; 12g) befestigt ist.

11. Füllnadel nach einem der Ansprüche 1 bis 10,
**dadurch gekennzeichnet,**
**dass** das Ventilelement (14; 14b; 14c; 14d; 14e; 14g) aus einem produktabweisenden Material ausgebildet ist oder auf der dem Füllnadelkörper (12; 12a; 12b; 12d; 12f; 12g) zugewandten Innenseite mit einer produktabweisenden Beschichtung versehen ist.

12. Fülleinrichtung (100) mit wenigstens einer Füllnadel (10; 10a bis 10g), die nach einem der Ansprüche 1 bis 11 ausgebildet ist, und mit Mitteln zum Rücksaugen von Flüssigkeit (1) in dem Füllnadelkörper (12; 12a; 12b; 12d; 12f; 12g).

## Claims

1. Filling needle (10; 10a to 10g) for dispensing a pharmaceutical liquid (1) into a container (2), comprising a preferably pin-shaped filling needle body (12; 12a; 12b; 12d; 12f; 12g) in which a product supply bore (13) is formed, and having at least one, preferably a plurality of product discharge bores (16; 16a to 16d) extending at least indirectly from the product supply bore (13) and opening in a region which, in relation to a longitudinal axis (18) of the filling needle body (12; 12a; 12b; 12d; 12f; 12g), is at a distance from an end (25; 25b; 25c) of the filling needle body, and having a valve element (14; 14b; 14c; 14d; 14e; 14g) covering the filling needle body (12; 12a; 12b; 12d; 12f; 12g) at least in the region of the at least one product discharge bore (16; 6a to 16d) and consisting of an elastic material, which valve element opens the at least one product discharge bore (16; 16a to 16d) beginning at a certain overpressure in the product discharge bore (16; 16a to 16d) for dispensing liquid (1) and closes the at least one product discharge bore (16; 16a to 16d) when the pressure falls below the certain overpressure.

2. Filling needle as claimed in claim 1,
**characterized in that**
the cross section of the filling needle body (12; 12a; 12b; 12d; 12f; 12g) is formed circular at least in the region of the at least one product discharge bore (16; 16a to 16d).

3. Filling needle as claimed in claim 1 or 2,
**characterized in that**,
when using a plurality of product discharge bores (16; 16a to 16d), the discharge openings thereof open in the outer wall of the filling needle body (12; 12a; 12b; 12d; 12f; 12g) at equal angular distances around the longitudinal axis (18).

4. Filling needle as claimed in one of claims 1 to 3,
**characterized in that**,
when using a plurality of product discharge bores (16b), the discharge openings thereof open in relation to the longitudinal axis (18) in at least two different regions in the outer wall of the filling needle body (12b).

5. Filling needle as claimed in one of claims 1 to 4,
**characterized in that**
the valve element (14; 14b) is in the form of a bushing and protrudes beyond the end (25; 25b) of the filling needle body when viewed in the direction of the longitudinal axis (18).

6. Filling needle as claimed in one of claims 1 to 4,
**characterized in that**
the valve element (14e; 14d) is in the form of a bushing, that the end (25c) of the filling needle body is formed conically and that the valve element (14c; 14d) ends in the conical region of the end (25c) of the filling needle body when viewed in the direction of the longitudinal axis (18).

7. Filling needle as claimed in one of claims 1 to 6,
**characterized in that**
the at least one discharge opening of a product discharge bore (16c) is formed in a region of reduced cross section (20c) of the filling needle body (12) radially surrounding the longitudinal axis (18).

8. Filling needle as claimed in claim 7,
**characterized in that**
the reduced cross section (20c) is formed as a circular groove (27).

9. Filling needle as claimed in one of claims 1 to 6,
**characterized in that**
the at least one product discharge bore (16a) is formed in a part (23) separate from the filling needle body (12) and serving as a closure for the filling needle body (12), that the discharge opening of the at least one product discharge bore (16a) is formed in the region of a reduced cross section (20a) of the part (23) radially surrounding the longitudinal axis (18) and that the reduced cross section (20a) has a rounded cross section.

10. Filling needle as claimed in one of claims 1 to 9,
**characterized in that**
the valve element (14; 14b; 14c; 14d; 14e; 14g) is attached to the filling needle body (12; 12a; 12b; 12d; 12f; 12g) by a separate fastening element (36; 36) or a positive connection.

11. Filling needle as claimed in one of claims 1 to 10,
**characterized in that**
valve element (14; 14b; 14c; 14d; 14e; 14g) is made of a product-repellent material or is provided with a product-repellent coating on its interior surface facing the filling needle body (12; 12a; 12b; 12d; 12f; 12g).

12. Filling device (100) having at least one filling needle (10; 10a to 10g) configured according to one of claims 1 to 11 and having means for drawing back liquid (1) in the filling needle body (12; 12a; 12b; 12d; 12f; 12g).

## Revendications

1. Aiguille de remplissage (10; 10a à 10g) destinée à délivrer un liquide pharmaceutique (1) dans un récipient (2), comprenant un corps d'aiguille de remplissage (12; 12a; 12b; 12d; 12f; 12g) de préférence en forme de broche dans lequel est ménagé un alésage d'alimentation en produit (13), avec au moins un, de préférence plusieurs alésages de sortie de produit (16; 16a à 16d) qui s'étendent au moins indirectement à partir de l'alésage d'alimentation en produit (13) et qui débouchent, sur une paroi extérieure du corps d'aiguille de remplissage (12; 12a; 12b; 12d; 12f; 12g), dans une zone qui est espacée d'une extrémité de corps d'aiguille de remplissage (25; 25b; 25c) par rapport à un axe longitudinal (18) du corps d'aiguille de remplissage (12; 12a; 12b; 12d; 12f; 12g), et avec un élément de soupape (14; 14b; 14c; 14d; 14e; 14g) qui recouvre le corps d'aiguille de remplissage (12; 12a; 12b; 12d ; 12f; 12g) au moins au niveau dudit au moins un alésage de sortie de produit (16; 16a à 16d) et qui est constitué d'un matériau élastique et qui libère ledit au moins un alésage de sortie de produit (16; 16a à 16d) pour délivrer du liquide (1) à partir d'une certaine surpression dans l'alésage de sortie de produit (16; 16a à 16d) et ferme ledit au moins un alésage de sortie de produit (16; 16a à 16d) lorsque la certaine surpression n'est pas atteinte.

2. Aiguille de remplissage selon la revendication 1,
**caractérisée par le fait**
**que** la section transversale du corps d'aiguille de remplissage (12; 12a; 12b; 12d; 12f; 12g) est réalisée de façon circulaire au moins au niveau dudit au moins un alésage de sortie de produit (16; 16a à 16d).

3. Aiguille de remplissage selon la revendication 1 ou 2,
**caractérisée par le fait**
**que**, lorsque plusieurs alésages de sortie de produit (16; 16a à 16d) sont utilisés, les sorties de ceux-ci débouchent à des intervalles angulaires réguliers autour de l'axe longitudinal (18) dans la paroi extérieure du corps d'aiguille de remplissage (12; 12a; 12b; 12d; 12f; 12g) .

4. Aiguille de remplissage selon l'une quelconque des revendications 1 à 3,
**caractérisée par le fait**
**que**, lorsque plusieurs alésages de sortie de produit (16b) sont utilisés, les sorties de ceux-ci débouchent dans au moins deux zones différentes dans la paroi extérieure du corps d'aiguille de remplissage (12b), par rapport à l'axe longitudinal (18).

5. Aiguille de remplissage selon l'une quelconque des revendications 1 à 4,
**caractérisée par le fait**
**que** ledit élément de soupape (14; 14b) est réalisé en forme de manchon et dépasse l'extrémité de corps d'aiguille de remplissage (25; 25b), vu dans la direction de l'axe longitudinal (18).

6. Aiguille de remplissage selon l'une quelconque des revendications 1 à 4,
**caractérisée par le fait**
**que** l'élément de soupape (14c; 14d) est réalisé en forme de manchon, que l'extrémité de corps d'aiguille de remplissage (25c) est réalisée de façon conique et que l'élément de soupape (14c; 14d), vu dans la direction de l'axe longitudinal (18), se termine dans la zone conique de l'extrémité de corps d'aiguille de remplissage (25c).

7. Aiguille de remplissage selon l'une quelconque des revendications 1 à 6,
**caractérisée par le fait**
**que** ladite au moins une sortie d'un alésage de sortie de produit (16c) est réalisée au niveau d'un rétrécissement de section transversale (20c) du corps d'aiguille de remplissage (12), qui s'étend radialement autour de l'axe longitudinal (18).

8. Aiguille de remplissage selon la revendication 7,
**caractérisée par le fait**
**que** ledit rétrécissement de section transversale (20c) est conçu sous la forme d'une rainure annulaire (27).

9. Aiguille de remplissage selon l'une quelconque des revendications 1 à 6,
**caractérisée par le fait**
**que** ledit au moins un alésage de sortie de produit (16a) est réalisé dans un composant (23) qui est séparé du corps d'aiguille de remplissage (12) et sert de fermeture pour le corps d'aiguille de remplissage (12), que la sortie dudit au moins un alésage de sortie de produit (16a) est réalisée au niveau d'un rétrécissement de section transversale (20a) du composant (23), qui s'étend autour de l'axe longitudinal (18), et que ledit rétrécissement de section transversale (20a) présente une section transversale arrondie.

10. Aiguille de remplissage selon l'une quelconque des revendications 1 à 9,
**caractérisée par le fait**
**que** ledit élément de soupape (14; 14b; 14c; 14d; 14e; 14g) est fixé au corps d'aiguille de remplissage (12; 12a; 12b; 12d; 12f; 12g) par un élément de fixation (36; 36) séparé ou par une liaison à engagement positif.

11. Aiguille de remplissage selon l'une quelconque des revendications 1 à 10,
**caractérisée par le fait**
**que** ledit élément de soupape (14; 14b; 14c; 14d; 14e; 14g) est fait d'un matériau répulsif de produit ou est pourvu d'un revêtement répulsif de produit sur la face intérieure montrant vers le corps d'aiguille de remplissage (12; 12a; 12b; 12d; 12f; 12g).

12. Dispositif de remplissage (100) comprenant au moins une aiguille de remplissage (10; 10a à 10g) qui est conçue selon l'une quelconque des revendications 1 à 11, et comprenant des moyens pour aspirer du liquide (1) dans le corps d'aiguille de remplissage (12; 12a; 12b; 12d ; 12f; 12g) .
